# EUROPEAN PATENT SPECIFICATION

(11) **EP 3 269 813 B1**
(45) Date of publication and mention of the grant of the patent: **24.05.2023**
(21) Application number: 16762023.6
(22) Date of filing: 11.03.2016
(51) Int. Cl.: C12N 15/10

(54) **A METHOD FOR ISOLATING A NUCLEIC ACID FROM AN FFPE TISSUE**
VERFAHREN ZUR ISOLIERUNG VON NUKLEINSÄUREN AUS FFPE-GEWEBE
PROCÉDÉ POUR ISOLER DES ACIDES NUCLÉIQUES DE TISSUE FFPE

(30) Priority: 12.03.2015 KR 20150034697
(43) Date of publication of application: 17.01.2018
(73) Proprietor: The Asan Foundation, Seoul 05505 (KR); University Of Ulsan Foundation For Industry Cooperation, Ulsan 44610 (KR)
(72) Inventor: JANG, Se Jin, Seongnam-si Gyeonggi-do 13517 (KR); CHUN, Sung Min, Seoul 05240 (KR); KIM, Tae Im, Seoul 05707 (KR); LEE, Jung Shin, Seoul 06214 (KR); CHOI, Eun Kyung, Seoul 05510 (KR)
(74) Representative: Gassner, Birgitta
(86) International application number: PCT/KR2016/002461
(87) International publication number: WO 2016/144136

(56) References cited:
- WO-A1-2007/068764
- WO-A1-2014/052551
- US-A1- 2005 208 510
- US-A1- 2006 078 923
- US-A1- 2006 141 450
- US-A1- 2013 053 254
- US-A1- 2014 364 597

## Description

### [Technical Field]

The present invention relates to a method for isolating a nucleic acid from a sample prepared by lysing a formalin-fixed, paraffin-embedded (FFPE) tissue fragment, a kit for isolating the nucleic acid, and a lysis buffer for the same.

### [Background Art]

Recently, molecular biological techniques have been used diversely in the medical field. In the molecular pathological aspect, in particular, those techniques enable pathological diagnosis from a morphological level to a gene level, and can also reveal genetic characteristics of a specific disease at the morphological and gene levels (DeMarzo et al., Lancet, (2003) 361, 955-64). For such diagnosis processes, hospitals collect a tissue in a formalin-fixed, paraffin-embedded (FFPE) form, which enables analysis and diagnosis of genetic information related to various clinical conditions (Roukos DH et al., Pharmacogenomics, (2010) 11,283-7). The patent application publication US2006/0078923 describes a method for isolating DNA from paraffin embedded tissues.

However, as a nucleic acid in the FFPE sample is broken down into small fragments and entangled with protein, there are limitations in the isolation of DNA from an FFPE tissue for clinical application at the molecular level (Feldman MY et al., Prog Nucleic Acid Res Mol Biol, (1973) 13, 1-49). Accordingly, the need for a method of isolating high-quality DNA having an optimized concentration and purity has been emphasized.

Kits, methods, *etc.* for isolating a nucleic acid from an FFPE tissue using chromatography are known (QIAamp FFPE tissue kit). However, there is still a demand for development of kits and methods for isolating a nucleic acid from an FFPE tissue which are improved compared to previously known methods.

### [Detailed Description]

### [Technical Problem]

The present inventors have made extensive efforts to develop a kit and a method for effectively obtaining a nucleic acid from an FFPE tissue, and as a result, have developed a sample by lysing an FFPE tissue fragment, and a method for obtaining a nucleic acid from magnetic beads to which the nucleic acid is bound, by adding a solution including a salt and PEG, and magnetic beads to the sample. They have also confirmed that the nucleic acid can be effectively isolated from the sample prepared by lysing the FFPE tissue fragment using the method developed above, thereby completing the present invention.

### [Technical Solution]

An object of the present invention is to provide a method for isolating a nucleic acid from a sample prepared by lysing a formalin-fixed, paraffin-embedded (FFPE) tissue fragment.

### [Advantageous Effect]

The method of the present invention for isolating a nucleic acid from an FFPE tissue has an effect of obtaining high yield of the nucleic acid from a sample prepared by lysing an FFPE tissue fragment in a short period of time.

### [Brief Description of Drawings]

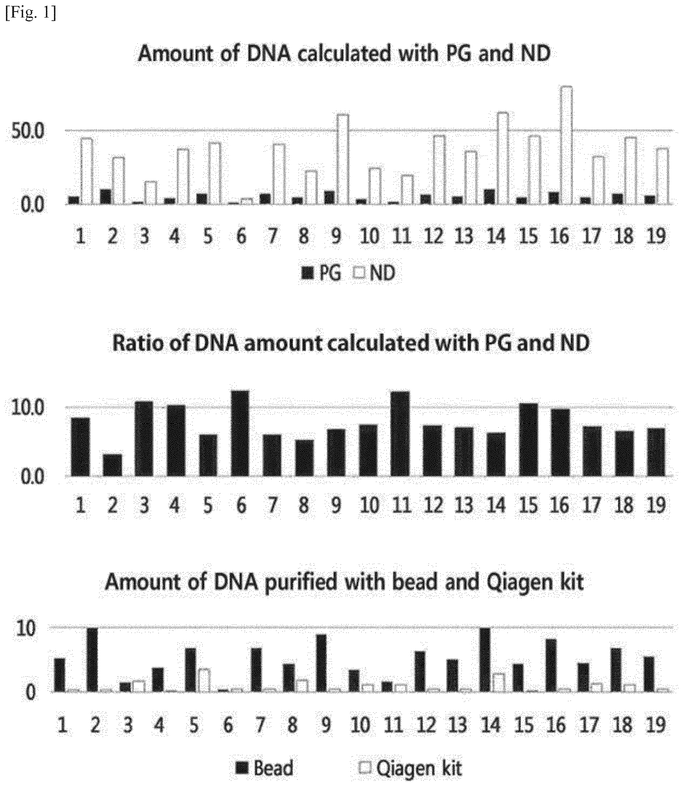

Fig. 1 shows the amount of DNA isolated from an aged FFPE tissue sample (2000 to 2004) by the bead method. Specifically, the DNA amount was measured by PicoGreen (PG) and NanoDrop (ND) methods. The graph on the top shows the amount of DNA (unit: µg), and that in the middle shows the DNA amount ratio of ND to PG. The graph on the bottom shows the amount of DNA (unit: µg) measured using PicoGreen after isolating the DNA from each sample using the beads or column (Qiagen kit).

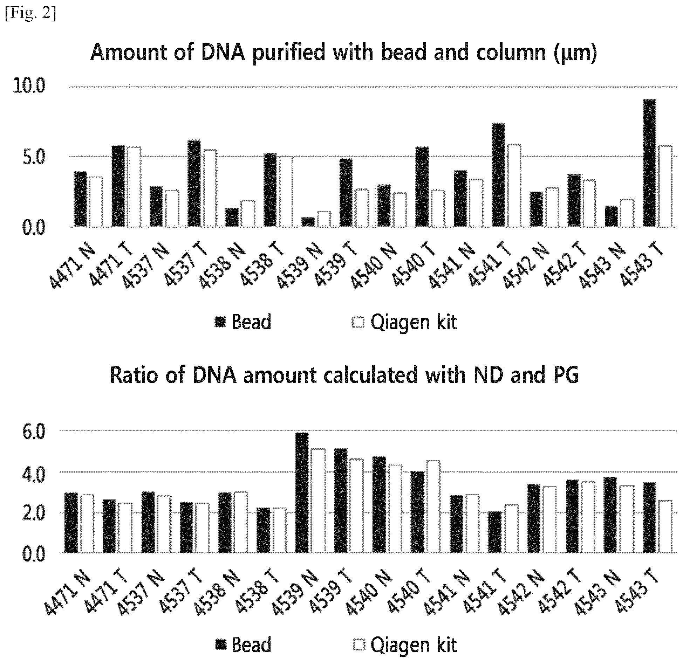

Fig. 2 shows comparison of the isolated DNA from a not-aged FFPE tissue fragment (2013) using the column and beads, measured by the PicoGreen (PG) and Nanodrop (ND) methods. The graph at the top shows the amount of the isolated DNA (unit: µg) measured by PG, and the graph on the bottom shows the DNA amount ratio of ND to PG.

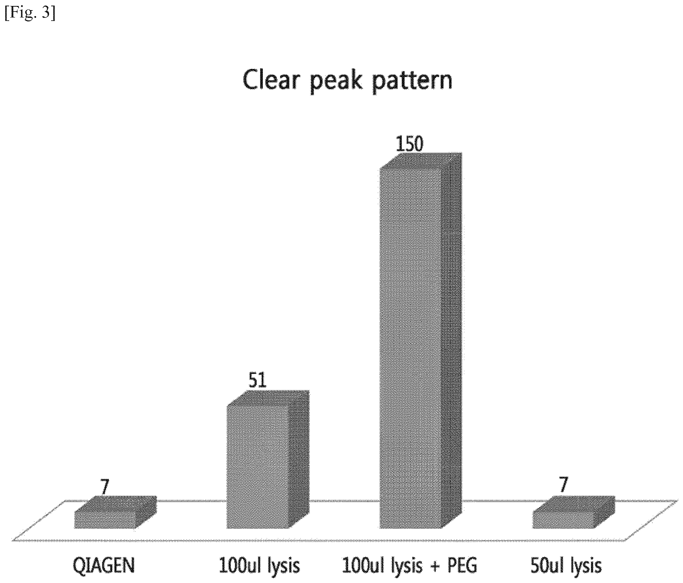

Fig. 3 shows qualitative analysis of clear peak pattern of 24 SNP site genotyping using 9 gNDA isolated from an FFPE tissue sample using a column (QIAGEN) or beads (100 µL lysis buffer, 100 µL lysis buffer + PEG, or 50 µL lysis buffer).

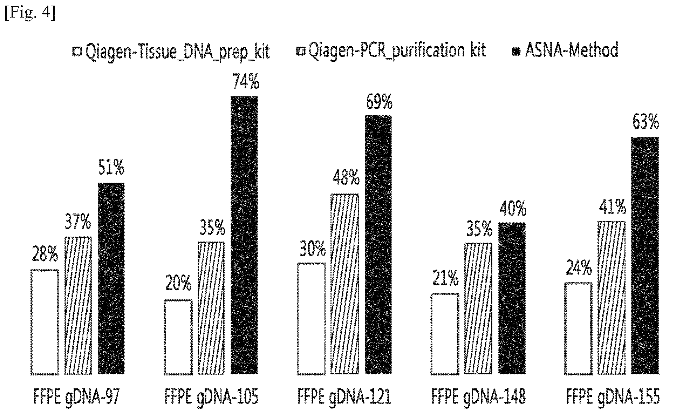

Fig. 4 shows comparison of the recovery ratio of 100 ng of gDNA isolated from 5 different FFPE tissues using QIAamp DNA FFPE Tissue kit when re-extracted using QIAamp DNA FFPE Tissue kit, QIAquick PCR Purification kit, or the bead method (ASAN-Method) of the present invention.

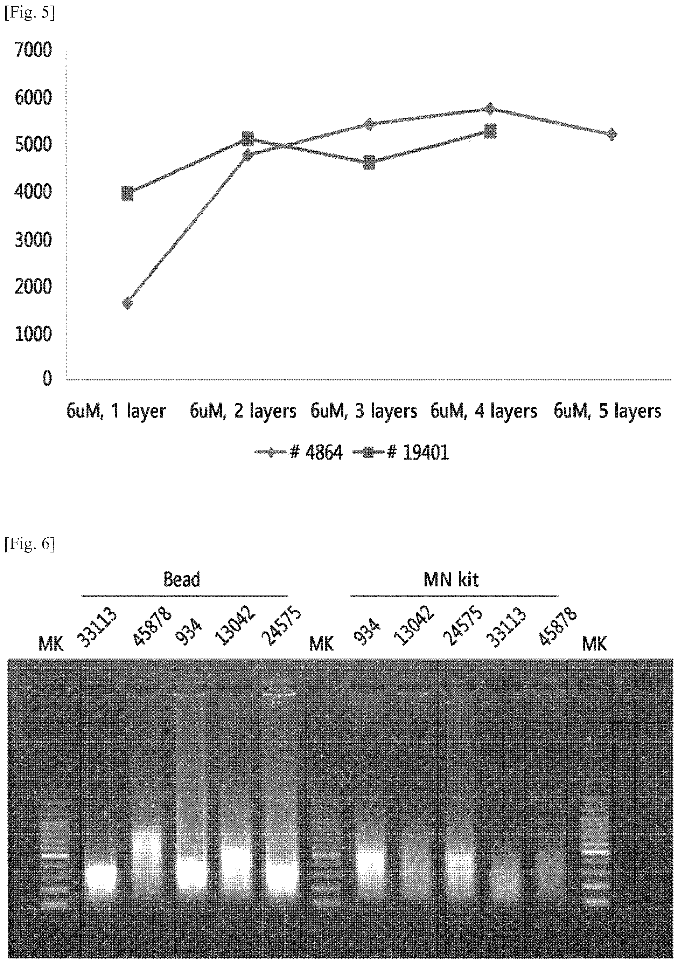

Fig. 5 shows the yield of DNA isolated from different amounts (6 µm thick, 1 layer to 5 layers) of tissue fragments of liver cancer FFPE tissue samples (#4864 and #19401) by the bead method. A size of the tissue included in the liver cancer FFPE block is 1 cm to 1.5 cm × 1 cm to 1.5 cm, and 100 µL of the elution buffer was used. The Y-axis on the graph represents the total amount (ng) of the obtained DNA.
Fig. 6 is an image of electrophoresis to confirm yields of the DNA isolated from 5 tissue samples using MN kit and the bead method of the present invention. The left 5 lanes (lanes 2 to 6) shows DNA isolated by the bead method of the present invention, and the right 5 lanes (lanes 8 to 12) shows DNA isolated by the MN kit. A total of 50 µL of the elution buffer was used, of which 5 µL was loaded on the gel. In order to confirm the objectivity of the experiment, researchers belonging to other institutions performed the experiment.

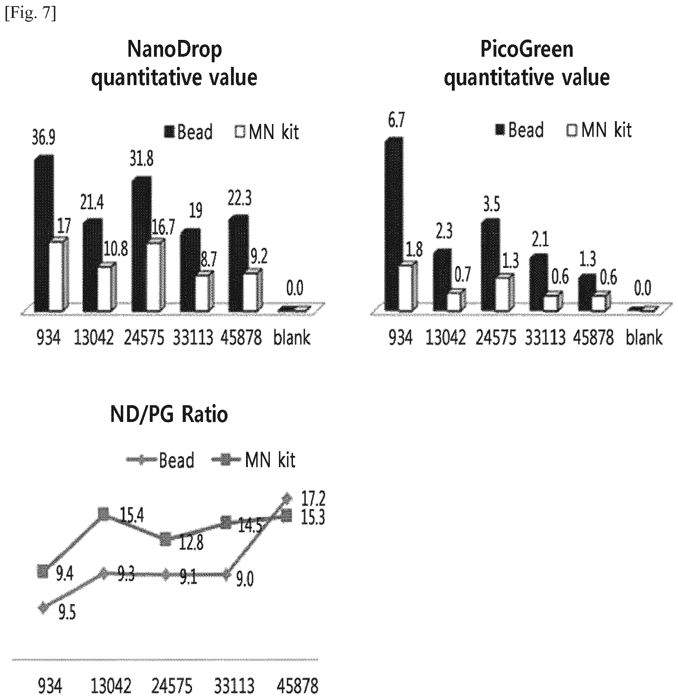

Fig. 7 shows the yield of DNA for 5 tissue samples, which is isolated by the bead method of the present invention and MN kit. A graph showing DNA amount (unit: µg) measured by NanoDrop (ND) is on the left and that by PicoGreen (PG) is in the middle, and a graph showing ND/PG ratio is on the right.

### [Best Mode]

A specific aspect of the present invention is a method for isolating a nucleic acid from a formalin-fixed, paraffin-embedded (FFPE) tissue, comprising:
(a) adding a solution comprising a 2.5 M of sodium chloride and 20% (w/v) polyethylene glycol (PEG-8000), and 100 µl magnetic bead solution to an isolated sample prepared by lysing an FFPE tissue fragment; and
(b) isolating the nucleic acid from the magnetic beads after obtaining the magnetic beads from the sample comprising the same.

As used herein, the term "formalin-fixed, paraffin-embedded (FFPE)" refers to a method for archiving or preserving a tissue isolated from an animal, and means fixing a tissue in formalin and embedding the fixed tissue in wax. Although not limited thereto, a specific process for FFPE tissue preparation may be as follows:
First, a tissue is isolated from an animal specimen by incision or biopsy. A fragment is prepared by incising the tissue, and the tissue is then fixed to prevent decomposition or disintegration and to be accurately examined under a microscope for pathological or cytological research. The fixing is a process of immobilizing, killing, and archiving for a purpose of staining and observing the tissue under a microscope. A further process makes the tissue permeable to a dye reagent, and by crosslinking with a very large molecule, stabilizes and traps the tissue at its position. For example, many fixatives such as a bouin solution, formaline, and liquid nitrogen are used for the purpose of the present invention. The fixed tissue is then embedded in wax and cut into thin sections, followed by staining with hematoxylin and eosin stain. Microtoming is performed by cutting the tissue into a microsection for research on staining with an antibody under a microscope.

With respect to methods for isolating a nucleic acid from the FFPE tissue, a conventional method is accompanied by problems such as DNA micro-fragmentation, entanglement with protein, *etc.,* thereby reducing yield. In this regard, there has been a demand for development of a method of obtaining a nucleic acid in high yield.

In the present invention, a method for isolating a nucleic acid bound to magnetic beads, by reacting a sample including the FFPE tissue fragment with magnetic beads and a solution including a salt and PEG, has been developed. In the case of using a solution including both a salt and PEG when reacting magnetic beads with a nucleic acid isolated from an FFPE tissue, it was confirmed that the magnetic beads can capture the nucleic acid more effectively, resulting in high yield of the nucleic acid. In particular, it was confirmed that a nucleic acid sample of an FFPE tissue fragment that had been manufactured at least 10 years prior could be effectively collected. Further, in regard to the method described above, the present inventors have developed a technology which enables effective isolation of a nucleic acid from an FFPE tissue with reproducibility by optimizing the constitution of the lysis buffer.

Hereinafter, steps and each constitution of the present invention will be described in more detail.

Step (a) of the method of the present invention binds a nucleic acid to magnetic beads by adding a solution, including 2.5 M of sodium chloride and 20% (w/v) PEG-8000, and magnetic beads to an isolated sample prepared by lysing an FFPE tissue fragment.

In the present invention, if the tissue is isolated from a subject whose nucleic acid need to be analyzed or obtained, no particular limitation is imposed on a type of the tissue as long as it is isolated from an animal. The FFPE tissue fragment, as an FFPE tissue having a size appropriate for nucleic acid isolation, may be in the form of a thin slice to have an appropriate size for the nucleic acid isolation. The thickness may be 2 µm to 10 µm, but is not limited thereto. The FFPE tissue for the method of the present invention for the nucleic acid isolation can be not only an FFPE tissue which has recently been manufactured but also one which has been archived at least for 10 years. Further, a conventional method for isolating a nucleic acid using beads generally includes addition of the beads after lysis and addition of ethanol, whereas the present invention has an advantage in that a nucleic acid can be isolated without adding ethanol between the lysis and the addition of beads.

The isolated sample prepared by lysing the FFPE tissue fragment may be prepared by lysing an FFPE tissue fragment with a lysis buffer.

Accordingly, the method of the present invention may include step 1 in which a lysis buffer is added to an FFPE tissue fragment; step 2 in which a solution including sodium chloride and PEG-8000 is added to the sample lysed in step 1; and step 3 in which magnetic beads are obtained from the sample where the magnetic beads are added, followed by isolating a nucleic acid therefrom, but is not limited thereto.

The present inventors established a constitution of the lysis buffer optimized for isolation of a nucleic acid from an FFPE sample with high purity and high yield, by comparing lysis buffers having various constitutions.

The lysis buffer used for the lysis of the FFPE tissue fragment may specifically include a chelating agent, an ionic surfactant, and Tris-Cl, but is not limited thereto.

According to an embodiment of the present invention, it was confirmed that use of a lysis buffer having the above constitution results in easier isolation of the magnetic beads and/or a higher yield compared to a case where a different lysis buffer was used.

In the present invention, the chelating agent can be used to isolate a divalent cation such as Mg²⁺ and Ca²⁺, and accordingly, can protect DNA from enzymes capable of disintegrating the DNA, but is not limited thereto.

An example of the chelating agent may be one selected from the group consisting of diethylenetriaminepentaacetic acid (DTPA), ethylenediaminetetraacetic acid (EDTA), ethylene glycol tetraacetic acid (EGTA), and N,N-bis(carboxymethyl)glycine (NTA), but is not limited thereto. EDTA was used as the chelating agent in one Example of the present invention.

The EDTA may be an EDTA part in EDTA compounds (*e.g.*, K₂EDTA, K₅EDTA, or Na₂EDTA), but is not limited thereto.

In the present invention, the ionic surfactant is a material having both hydrophilic and hydrophobic parts in a single molecule, and shows ionic characteristics during dissociation.

Examples of the ionic surfactant which can be used in the present invention include sodium dodecyl sulfate (SDS), ammonium lauryl sulfate, sodium lauryl ether sulfate (SLES), sodium myreth sulfate, dioctyl sodium sulfosuccinate, perfluorooctanesulfonate (PFOS), perfluorobutanesulfonate, linear alkylbenzene sulfonates (LABs), sodium lauroyl sarcosinate, perfluorononanoate (PFOA), and perfluorooctanoate (PFO), but is not limited thereto.

The lysis buffer includes the chelating agent in a concentration of 10 mM to 100 mM; the ionic surfactant in a concentration of 0.1% (w/v) to 5% (w/v); and/or the Tris-Cl in a concentration of 10 mM to 100 mM, but is not limited thereto.

A pH of the lysis buffer may be 7.0 to 8.5.

More specifically, the lysis buffer may include EDTA, SDS, and Tris-Cl, but is not limited thereto.

Additionally, when lysing an FFPE tissue sample with the lysis buffer, a proteinase can be added as well.

Various proteinases conventionally used for nucleic acid isolation can be used; for example, proteinase K can be used.

In the present invention, the addition of the magnetic beads and the solution including a sodium chloride and PEG-8000 to the sample can be performed sequentially or simultaneously, but is not limited thereto.

In the case of performing the addition sequentially, the solution can be added after the addition of magnetic beads, or the magnetic beads can be added after the addition of the solution.

In the present invention, the solution including sodium chloride and PEG-8000 added along with the magnetic beads is not particularly limited, but may aid in capturing a small nucleic acid fragment of 100 bp or 100 nt or below.

A type of the salt in the solution including the salt and polyethylene glycol (PEG) is not particularly limited, but may be sodium chloride as an example.

An average molecular weight of PEG of the solution may be 6,000 Da to 10,000 Da, but is not limited thereto.

More specifically, the solution including the salt and PEG may include the salt in a concentration of 1 M to 4 M and the PEG in a concentration of 10% (w/v) to 40% (w/v), but is not limited thereto.

The magnetic beads in the present invention refer to particles or beads which react to a magnetic field. Generally, magnetic beads do not have a magnetic field, but refer to a material forming a magnetic dipole when exposed to a magnetic field; for example, a material which can be magnetized under a magnetic field but which does not have magnetism itself in the absence of the magnetic field. The magnetism used in the present invention includes both paramagnetic and superparamagnetic materials, but is not particularly limited.

For the purpose of the present invention, it is preferable that the magnetic beads have a characteristic of binding to a nucleic acid; for example, the magnetic beads are in the form of having a functional group (*e.g.,* -COOH) which binds to the nucleic acid, but the beads are not limited thereto.

The method of the present invention for isolating a nucleic acid from an FFPE tissue is not particularly limited, but may include deparaffinization of the FFPE tissue.

The FFPE tissue is a fixed tissue embedded in wax. As most embedded media are hydrophobic, removal of inactive materials may be necessary before histological, cytological, or molecular biological analysis that mostly uses a hydrophilic reagent. As used herein, the term "deparaffinization" or "dewaxing" refers to removing a part or whole part of any type of an embedded medium from a biological sample and is widely used in the present invention. For example, although not limited, a paraffin-embedded tissue fragment may be deparaffinized by treatment with an organic solvent, e.g., toluene, xylene, limonene, or other appropriate solvents.

According to one embodiment of the present invention, deparaffinization was performed by repeatedly performing addition of xylene and washing with ethanol.

In step (b) of the present invention, the magnetic beads are obtained from a sample to which the magnetic beads are added, and a nucleic acid is isolated from the magnetic beads.

Specifically, step (b) isolates the nucleic acid attached to the magnetic beads added in step (a) from the magnetic beads.

Before obtaining the magnetic beads from the sample to which the magnetic beads are added, a step of washing the magnetic beads may be included.

The magnetic bead wash can be performed using a 50% (v/v) to 95% (v/v) ethanol solution, specifically an 80% (v/v) to 90% (v/v) ethanol solution.

The washing can be performed by placing the magnetic beads under the magnetic stand and collecting the magnetic beads, followed by removing a supernatant and adding a wash buffer. Additionally, such washing can be performed at least once.

After optionally performing the washing, the nucleic acid can be isolated from the magnetic beads by isolating the magnetic beads, adding an elution buffer to the magnetic beads, *etc.*

The following aspect is not according to the invention and is present for illustration purposes only. Described herein is a kit for isolating a nucleic acid from an FFPE tissue, comprising a lysis buffer, a solution including a salt and polyethylene glycol (PEG), and magnetic beads.

The lysis buffer, the solution including a salt and PEG, and the magnetic beads are the same as explained above.

Additionally, the kit may further include other apparatuses, solutions, *etc.* generally used for nucleic acid isolation; an instruction for the nucleic acid isolation from an FFPE tissue; *etc.,* but is not limited thereto.

Additionally, the kit may further include a wash buffer for magnetic beads and an apparatus for isolating magnetic beads (e.g., magnetic stand), but is not limited thereto.

Further described herein is a lysis buffer for isolating a nucleic acid from an FFPE tissue, including a chelating agent, an ionic surfactant, and Tris-Cl.

The chelating agent, ionic surfactant, and lysis buffer for isolating a nucleic acid from an FFPE are the same as explained above.

### [Mode for Invention]

Hereinafter, the present invention will be described in more detail with reference to the following examples and experimental examples.

### Example 1: Isolating genome DNA from an FFPE tissue sample (including paraffin removal)

### (1) FFPE block fragmentation and paraffin removal

The FFPE block was cut to a thickness of 6 µm, and one or two FFPE tissue fragments were then placed in a 1.5 mL tube. 1 mL of xylene was added thereto and mixed, and then was centrifuged at 12,000× g for 3 minutes at room temperature. After removing the supernatant, 0.8 mL of xylene and 0.4 mL of EtOH were added and mixed, followed by centrifuging at 12,000× g for 3 minutes at room temperature and removing the supernatant. 1 mL of EtOH was added and mixed again and was centrifuged at 12,000× g for 3 minutes at room temperature, and the supernatant was removed. In order to completely evaporate the remaining EtOH, incubation was performed for 0.5 hours to 1 hour at room temperature.

### (2) Lysis and genome DNA isolation

90 µL of a lysis buffer (10 mM Tris-Cl, pH 8.0, 100 mM EDTA, pH 8.0, 0.5% SDS) and 10 µL of proteinase K were added to the tube including a paraffin-removed FFPE tissue fragment and mixed, and then incubated overnight at 56°C.

Next, 100 µL of the magnetic beads (AMPure XP) and 100 µL of solution A (2.5 MNaCl, 20% PEG-8000) were added to the tube and mixed. The tube was then incubated for 3 minutes at room temperature. The tube was incubated on a magnetic stand for 5 minutes, and a supernatant was removed.

After adding and reacting 500 µL of wash buffer (85% EtOH), an ethanol supernatant was removed on the magnetic stand. This washing process was repeated several times.

The beads were dried and taken out of the reaction tube, and 60 µL of a lysis buffer (10 mM Tris-Cl, pH 8.5, or tertiary distilled water) was added to the reaction tube. The reaction tube was placed on the magnetic stand, and an eluate was isolated, thereby obtaining a genome DNA sample.

### Example 2: Isolation of genome DNA from an FFPE tissue sample (excluding paraffin removal)

### (1) FFPE block fragmentation

The FFPE block was cut to a thickness of 6 µm, and one or two FFPE tissue fragments were then placed in a 1.5 mL tube.

### (2) Lysis and genome DNA isolation

200 µL of a lysis buffer (10 mM Tris-Cl, pH 8.0, 100 mM EDTA, pH 8.0, 0.5% SDS), mineral oil, and 10 L of proteinase K were added to the tube including an FFPE tissue fragment and mixed, and then incubated overnight at 56°C.

Next, a lower phase was separated and transferred to a new tube. 100 µL of the magnetic beads (AMPure XP) and 100 µL of solution A (2.5 M NaCl, 20% PEG-8000) were added to the tube and mixed. The tube was then incubated for 3 minutes at room temperature. The tube was incubated on a magnetic stand for 5 minutes, and a supernatant was removed.

After adding and reacting 500 µL of wash buffer (85% EtOH), an ethanol supernatant was removed on the magnetic stand. This washing process was repeated several times.

The beads were dried and taken out of the reaction tube, and 60 µL of a lysis buffer (10 mM Tris-Cl, pH 8.5, or tertiary distilled water) was added to the reaction tube. The reaction tube was placed in the magnetic stand, and an eluate was isolated, thereby obtaining a genome DNA sample.

After adding and reacting 500 µL of wash buffer (85% EtOH), an ethanol supernatant was removed on the magnetic stand. This washing process was repeated several times.

The beads were dried and taken out of the reaction tube, and 60 µL of a lysis buffer (10 mM Tris-Cl, pH 8.5, or tertiary distilled water) was added to the reaction tube. The reaction tube was placed on the magnetic stand, and an eluate was isolated, thereby obtaining a genome DNA sample.

When genome DNA extracted by the method (the paraffin-removing process included) of Example 1 and that extracted by the method (the paraffin-removing process excluded) of Example 2 are compared, quality-related characteristics of the DNA were similar, but yield of the method in which the paraffin-removing process is excluded was about 80% to 90% of that of the method in which the paraffin-removing process is included. Experiments would be more convenient if the deparaffinization is not additionally performed, and could be used very effectively for genome DNA isolation from several samples. All experimental procedures hereinafter were performed by a method including the deparaffinization of Example 1.

### Example 3: Quantitative analysis of DNA

### (1) Quantitation using NanoDrop (ND)

NanoDrop 2000 (Thermo Scientific) was used in the dsDNA concentration measurement mode. After measuring a blank value using a DNA extract eluate as a reference, absorbance at OD₂₆₀ was measured using 1 µL of the extracted DNA, and a concentration was calculated using the following formula: dsDNA concentration = Absorbance (OD₂₆₀) ^{∗} 50 ng/µL. A final yield was calculated by multiplying 100 µL (a total volume of the extracted solvent).

### (2) Quantitation using PicoGreen (PG)

Quant-Ti PicoGreen dsDNA assay kit (Invitrogen) was purchased to use to measure dsDNA concentrations.

### A. Preparation of PicoGreen reagent

30 minutes before measuring concentrations, a PicoGreen reagent was diluted to 1/200 with a 1× TE buffer, and was stored wrapped in aluminum foil to protect from light at room temperature.

### B. Preparation of DNA of normal concentration (Lambda DNA)

A two-fold serial dilution of DNA in a standard concentration (100 ng/µL) was performed using 1× TE buffer to give 50 ng/µL, 25 ng/µL, 12.5 ng/µL, 6.3 ng/µL, 3.2 ng/µL, 1.6 ng/µL, and 0.8 ng/µL. 150 µL of 1× TE buffer was added to 3 µL of each of the above standard-concentration DNA dilutions prepared to have the concentrations of 50 ng/µL to 0.8 ng/µL and mixed well, and the mixtures were then stored.

### C. Preparation of extracted FFPE DNA

30 minutes before measuring concentrations, 3 µL of the extracted FFPE DNA was mixed well with 150 µL of 1× TE buffer and stored.

### D. Concentration measurement and final yield calculation

50 µL of the PicoGreen reagent prepared in the above A. along with 50 µL of each of the DNA (the serially diluted standard-concentration DNA and the extract FFPE DNA sample prepared in B. and C.) were added to each well of 96-well assay plates having no light transmission and were mixed well, followed by storage for 3 minutes at room temperature.

Next, the mixture was centrifuged at 2000× g for 1 minute, and was excited at a wavelength of 485 nm. A value was measured at a wavelength of 535 nm. For high accuracy of the measured value, the experiments on all DNA were repeated three times, and a value for a negative control was measured using 1 × TE in which DNA is not included. A calibrated value was calculated by subtracting the measured value of the negative control from measured values of all DNA.

Using the concentrations of the serially diluted standard-concentration DNA and the calibrated value of the wavelength of 535 nm, an equation for DNA concentration according to measured values at 535 nm was prepared. A p-value (for linearity of the DNA concentrations and the measured values at 535 nm) of the equation was measured to confirm whether it was at least 0.99. If the value was below 0.99, the p-value was measured again to prepare a standard concentration equation.

The calibrated value was applied to the standard concentration equation to calculate each DNA concentration, and 100 µL, a total volume of the extracted solvent, was multiplied with each DNA concentration to calculate a final yield.

### Example 4: Preparation using an FFPE tissue aged for at least 10 years

As an aged FFPE block undergoes a high degree of gDNA fragmentation, DNA yield may be low in a case of a kit manufactured for isolating genome DNA in a general size. In the present research, a solution consisting of PEG and NaCl having an optimized concentration was added during the DNA extraction process so as to extract DNA fragmented even in a small size, resulting in a significantly increased yield of the aged FFPE DNA.

As shown in the graphs at the top and the middle of Fig. 1, the reason for the large difference in the quantitative value between ND and PG is that the DNA isolation method of the present invention enables small-size genome DNA to be obtained from DNA fragmentation. As shown in the bottom graph, it was confirmed that when fragmented DNA was extracted in a small size from the aged FFPE block, use of the bead method of the present research resulted in a 10-fold higher yield compared to the QIAGEN kit.

### Example 5: Preparation using an unaged FFPE tissue

In a case of an FFPE block manufactured recently, a relatively large size of gDNA can be obtained as DNA fragmentation has not occurred extensively. Accordingly, it was confirmed that the DNA yield and the quantitative ratio of ND to PG were similar when the bead method and QIAGEN kit were used (Fig. 2).

### Example 6: Clear genotyping of the sample obtained by the genome DNA PREP method of Example 1

In order to evaluate the obtained genome DNA quality, clear genotyping was performed. Specifically, 9 genome DNAs isolated from an FFPE tissue sample by the column method (QIAGEN) or the bead method (100 µL lysis buffer, 100 µL lysis buffer + PEG or 50 µL lysis buffer) were used to perform genotyping of 24 SNP sites, and quantitative analysis of a clear peak pattern was performed. The condition of 100 µL lysis buffer + PEG of the bead method refers to using 100 µL of the lysis buffer for lysis, followed by adding 100 µL of solution A containing a PEG and NaCl mixture, whereas the other condition of 100 µL lysis buffer and 50 µL lysis buffer refers to using only the lysis buffer but not adding solution A.

As the above analysis is analysis of SNP, *i.e.,* a Germline mutation, the SNP can be analyzed as only being either a homologous base, homo (100%), or a heterologous base, hetero (50%). However, depending on the quality of genome DNA used as a template, there is a case where a hetero with a pattern making difficult to determine whether it is homo or hetero may result, *i.e.,* 80% to 90% homology for homo, 10% to 20% or 80% to 90% homology for hetero, and thus the quality of the extracted DNA cannot be determined by the above analysis.

Having the genotype confirmed using Sanger sequencing as a gold standard, the genotyping was performed on 24 SNPs of the genome DNA obtained by each method, and was analyzed with regard to which method resulted in the most accurate analysis on DNA, *i.e.,* whether it is homo (100%) or hetero (50%). As 24 SNPs for 9 genome DNA were analyzed, a number of the most accurate genotypes out of total of 216 genotypes was counted, and which method resulted in the greatest number of accurate genotypes was analyzed. Details of the experimental method are as follows:

### (1) Designing primers for 24 SNP sites

Using an Assay designer module of Tyler program (Sequenom), PCR and UEP primers were designed for each of 24 SNP sites. A size of an amplicon was about 100 bp, and a location of the SNPs was designed to be in the middle of each amplified product. The designed primers (SEQ ID NOS: 1 to 120) were as shown in Tables 1 and 2 below.

**[Table 1]**

| PCR Primer Information | | | |
|---|---|---|---|
| SNP_ID | 2nd-PCRP | 1st-PCRP | AMP _LEN |
| rs248205 | ACGTTGGATGGGCTCATGGGA AGAATCATC (SEQ ID NO: 1) | ACGTTGGATGGGGCAACATA CCACTATTG (SEQ ID NO: 2) | 102 |
| rs2051068 | ACGTTGGATGACCCTGGAAAC CATTTCAGA (SEQ ID NO: 3) | ACGTTGGATGCTGAAGGTAA AGATTCAAG (SEQ ID NO: 4) | 101 |
| rs1950501 | ACGTTGGATGAATGACTCACC ACTGACCAC (SEQ ID NO: 5) | ACGTTGGATGTGTTGTCTGG GAACTCAGGG (SEQ ID NO: 6) | 104 |
| rs1952966 | ACGTTGGATGGGCTCTGGTTA CAACAGCTT (SEQ ID NO: 7) | ACGTTGGATGAGAAGTTTGC TTGGCTGAAG (SEQ ID NO: 8) | 120 |
| AMG_mid1 00 | ACGTTGGATGGGCTTGAGGCC AACCATCAG (SEQ ID NO: 9) | ACGTTGGATGCCTCATCCTG GGCACCCTGG (SEQ ID NO: 10) | 99 |
| rs1385306 | ACGTTGGATGGTCTGTCAGCT GTTCTGATG (SEQ ID NO: 11) | ACGTTGGATGGGCTCTGTAT AGAGCTTTGG (SEQ ID NO: 12) | 99 |
| rs1365740 | ACGTTGGATGGTACTTACCTC CTGAGGTAG (SEQ ID NO: 13) | ACGTTGGATGACTTCCTATG TTGCCAGCAC (SEQ ID NO: 14) | 102 |
| rs2016207 | ACGTTGGATGCTGATGCAAAA ATCTATGGC (SEQ ID NO: 15) | ACGTTGGATGGCTCTAGTAG ATTGCTAGCC (SEQ ID NO: 16) | 107 |
| rs1571256 | ACGTTGGATGATACCGCAGAA GTTTGGCAC (SEQ ID NO: 17) | ACGTTGGATGGCATTCTAAA CATGCCTCTC (SEQ ID NO: 18) | 99 |
| rs1350836 | ACGTTGGATGATTTCAGACTG TTGTGCTGG (SEQ ID NO: 19) | ACGTTGGATGCCTGTGGCCT TTTCATGGAG (SEQ ID NO: 20) | 102 |
| rs120434 | ACGTTGGATGATGCCTTGTTC CATGTGCTG (SEQ ID NO: 21) | ACGTTGGATGACACAAGTGG AAGCTTGCAG (SEQ ID NO: 22) | 101 |
| rs2347790 | ACGTTGGATGATGCTCCACCC ACACAGTTC (SEQ ID NO: 23) | ACGTTGGATGACCTATGCAA CAGCTGGAAG (SEQ ID NO: 24) | 93 |
| rs298898 | ACGTTGGATGCCATATATACA GGGTGTATAG (SEQ ID NO: 25) | ACGTTGGATGATAGTGTAGT GAGTGCTATG (SEQ ID NO: 26) | 103 |
| rs2380657 | ACGTTGGATGGCTAGGGTTGA AAACCAATG (SEQ ID NO: 27) | ACGTTGGATGGAATATGAGC ACAACACACG (SEQ ID NO: 28) | 110 |
| rs2180770 | ACGTTGGATGCACTTGTCACA AACTCTAGG (SEQ ID NO: 29) | ACGTTGGATGAACCACAGTG AGCATGGAAG (SEQ ID NO: 30) | 113 |
| rs58616 | ACGTTGGATGTAGGCAGAAAA GGGCTGAAG (SEQ ID NO: 31) | ACGTTGGATGCCCTTCTGCTT TAACACTATC (SEQ ID NO: 32) | 114 |
| rs265005 | ACGTTGGATGATCAGGCACAA TCTCTACCC (SEQ ID NO: 33) | ACGTTGGATGACACAAAGCT ACCACTGCAC (SEQ ID NO: 34) | 101 |
| rs1259859 | ACGTTGGATGGCCAATTTTAT AGAAACTC (SEQ ID NO: 35) | ACGTTGGATGTAGTATCAAG GTTTTCTGGC (SEQ ID NO: 36) | 104 |
| rs1549944 | ACGTTGGATGTGTCACATATG CTGGCCTTG (SEQ ID NO: 37) | ACGTTGGATGCTCTCTTGAA GAAGGTGCAG (SEQ ID NO: 38) | 100 |
| rs1028330 | ACGTTGGATGTTTTCGGGCAG TGAAGAGAC (SEQ ID NO: 39) | ACGTTGGATGCTCTACATCT GTGGGTCTAG (SEQ ID NO: 40) | 105 |
| rs1390272 | ACGTTGGATGCCTTTGATATTT GTTCCAC (SEQ ID NO: 41) | ACGTTGGATGCAGGATAGTC TACTATGTGC (SEQ ID NO: 42) | 97 |
| rs1434199 | ACGTTGGATGGGCATAGGGAG | ACGTTGGATGCACCTCTGCC | 111 |
| | CTGAATCAA (SEQ ID NO: 43) | CCCTAATTTC (SEQ ID NO: 44) | |
| rs464221 | ACGTTGGATGTGAGGACTTGG GATTAGGAC (SEQ ID NO: 45) | ACGTTGGATGAGGCAGCAGC AGAAGTTTAG (SEQ ID NO: 46) | 87 |
| rs1522307 | ACGTTGGATGTAGGGTCCAGA AATGTGTTG (SEQ ID NO: 47) | ACGTTGGATGGGAGAAGCAA GCCATAGATG (SEQ ID NO: 48) | 95 |

**[Table 2]**

| UEP Primer Information | | | | | | | | |
|---|---|---|---|---|---|---|---|---|
| SNP_ ID | UEP _M ASS | UEP_SEQ | EXT 1_C ALL | EXT 1_M ASS | EXT1_SEQ | EXT 2_C ALL | EXT 2_M ASS | EXT2_SEQ |
| rs248 205 | 4803 | AAAGCTCCAA CACACT (SEQ ID NO: 49) | C | 5050 | AAAGCTCCAA CACACTC (SEQ ID NO: 50) | G | 5090 | AAAGCTCCAA CACACTG (SEQ ID NO: 51) |
| rs205 1068 | 4955 | GAGCGCACAG GTATAG (SEQ ID NO: 52) | T | 5226 | GAGCGCACAG GTATAGA (SEQ ID NO: 53) | C | 5243 | GAGCGCACAG GTATAGG (SEQ ID NO: 54) |
| rs195 0501 | 5284 | GTGGGTACAA AGGTCAA (SEQ ID NO: 55) | G | 5531 | GTGGGTACAA AGGTCAAC (SEQ ID NO: 56) | C | 5571 | GTGGGTACAA AGGTCAAG (SEQ ID NO: 57) |
| rs195 2966 | 5194 | CTTGGCTGAA GCAATAC (SEQ ID NO: 58) | A | 5466 | CTTGGCTGAA GCAATACA (SEQ ID NO: 59) | G | 5482 | CTTGGCTGAA GCAATACG (SEQ ID NO: 60) |
| AMG _mid 100 | 5203 | tGGACCACTTG AGAAAC (SEQ ID NO: 61) | G | 5451 | tGGACCACTTG AGAAACC (SEQ ID NO: 62) | T | 5475 | tGGACCACTTG AGAAACA (SEQ ID NO: 63) |
| rs138 5306 | 5423 | TTTGGCTCATC TGTTCTC (SEQ ID NO: 64) | G | 5670 | TTTGGCTCATC TGTTCTCC (SEQ ID NO: 65) | C | 5710 | TTTGGCTCATC TGTTCTCG (SEQ ID NO: 66) |
| rs136 | 5631 | CACCTTTTTCC | G | 5918 | CACCTTTTTCC | T | 5958 | CACCTTTTTCC |
| 5740 | | TCTTCATT (SEQ ID NO: 67) | | | TCTTCATTG (SEQ ID NO: 68) | | | TCTTCATTT (SEQ ID NO: 69) |
| rs201 6207 | 5805 | AGCACAGAAT CCTTTAGAA (SEQ ID NO: 70) | C | 6052 | AGCACAGAAT CCTTTAGAAC (SEQ ID NO: 71) | T | 6132 | AGCACAGAAT CCTTTAGAAT (SEQ ID NO: 72) |
| rs157 1256 | 6019 | TGCCTCTCAG TACTCTAGTC (SEQ ID NO: 73) | T | 6290 | TGCCTCTCAGT ACTCTAGTCA (SEQ ID NO: 74) | C | 6306 | TGCCTCTCAGT ACTCTAGTCG (SEQ ID NO: 75) |
| rs135 0836 | 6094 | CCACCTCAAA GGGATATTAA (SEQ ID NO: 76) | C | 6341 | CCACCTCAAA GGGATATTAA C (SEQ ID NO: 77) | T | 6421 | CCACCTCAAA GGGATATTAA T (SEQ ID NO: 78) |
| rs120 434 | 6237 | ggGTTGGCAAC ATGGTTAAG (SEQ ID NO: 79) | C | 6484 | ggGTTGGCAAC ATGGTTAAGC (SEQ ID NO: 80) | G | 6524 | ggGTTGGCAAC ATGGTTAAGG (SEQ ID NO: 81) |
| rs234 7790 | 6373 | ccccGATGTGC GTATGCCTTA (SEQ ID NO: 82) | A | 6644 | ccccGATGTGCG TATGCCTTAA (SEQ ID NO: 83) | G | 6660 | ccccGATGTGCG TATGCCTTAG (SEQ ID NO: 84) |
| rs298 898 | 6451 | GTGAGTGCTA TGATCATTAT C (SEQ ID NO: 85) | C | 6698 | GTGAGTGCTAT GATCATTATCC (SEQ ID NO: 86) | T | 6778 | GTGAGTGCTA TGATCATTATC T (SEQ ID NO: 87) |
| rs238 0657 | 6733 | tggaACAACAC ACGTTTTTAGT (SEQ ID NO: 88) | C | 6981 | tggaACAACACA CGTTTTTAGTC (SEQ ID NO: 89) | T | 7061 | tggaACAACACA CGTTTTTAGTT (SEQ ID NO: 90) |
| rs218 0770 | 6943 | ccAAATATAAC CTTGATCCTCT G (SEQ ID NO: 91) | T | 7214 | ccAAATATAAC CTTGATCCTCT GA (SEQ ID NO: 92) | C | 7230 | ccAAATATAAC CTTGATCCTCT GG (SEQ ID NO: 93) |
| rs586 16 | 7014 | TCTGCTTTAAC ACTATCAGGG TA (SEQ ID | G | 7261 | TCTGCTTTAAC ACTATCAGGG TAC (SEQ ID | A | 7341 | TCTGCTTTAAC ACTATCAGGG TAT (SEQ ID |
| | | NO: 94) | | | NO: 95) | | | NO: 96) |
| rs265 005 | 7022 | tttaTGCTCAAC TGGACTATAA A (SEQ ID NO: 97) | T | 7293 | tttaTGCTCAACT GGACTATAAA A (SEQ ID NO: 98) | C | 7309 | tttaTGCTCAACT GGACTATAAA G (SEQ ID NO: 99) |
| rs125 9859 | 7110 | gatgGGAGGTG ATCCTTCTTAT A (SEQ ID NO: 100) | C | 7397 | gatgGGAGGTGA TCCTTCTTATA G (SEQ ID NO: 101) | A | 7437 | gatgGGAGGTGA TCCTTCTTATA T (SEQ ID NO: 102) |
| rs154 9944 | 7469 | gggggAGGAAAG AAACACTGAT CCA (SEQ ID NO: 103) | A | 7740 | ggggAGGAAAG AAACACTGAT CCAA (SEQ ID NO: 104) | G | 7756 | gggggAGGAAAG AAACACTGAT CCAG (SEQ ID NO: 105) |
| rs102 8330 | 7561 | ccacTGACATGT ATCCACCATT ATG (SEQ ID NO: 106) | T | 7832 | ccacTGACATGT ATCCACCATTA TGA (SEQ ID NO: 107) | C | 7848 | ccacTGACATGT ATCCACCATTA TGG (SEQ ID NO: 108) |
| rs139 0272 | 7613 | gCTTGGTTTTT CTTAACAAGT TCAC (SEQ ID NO: 109) | T | 7884 | gCTTGGTTTTT CTTAACAAGTT CACA (SEQ ID NO: 110) | C | 7900 | gCTTGGTTTTT CTTAACAAGTT CACG (SEQ ID NO: 111) |
| rs143 4199 | 7686 | gATTTGAAGG TCTAGAACTT TCATT (SEQ ID NO: 112) | G | 7933 | gATTTGAAGGT CTAGAACTTTC ATTC (SEQ ID NO: 113) | C | 7973 | gATTTGAAGGT CTAGAACTTTC ATTG (SEQ ID NO: 114) |
| rs464 221 | 8027 | gtCAGCAGCAG AAGTTTAGAC AATTA (SEQ ID NO: 115) | G | 8274 | gtCAGCAGCAG AAGTTTAGAC AATTAC (SEQ ID NO: 116) | A | 8354 | gtCAGCAGCAG AAGTTTAGAC AATTAT (SEQ ID NO: 117) |
| rs152 2307 | 8069 | tagaAAGCCATA GATGAAAATA CGGA (SEQ ID | C | 8317 | tagaAAGCCATA GATGAAAATA CGGAC (SEQ ID | T | 8396 | tagaAAGCCATA GATGAAAATA CGGAT (SEQ ID |
| | | NO: 118) | | | NO: 119) | | | NO: 120) |

### (2) iPLEX reaction (using Sequenom's iPLEX kit)

### A. Multiplex PCR reaction

5 ng of DNA extracted by each method, 1.2 µL of a mixture of the primers (0.5 µM each) of Table 1, 0.1 µL of 25 mM dNTP mix, 0.12 µL of 5 U/µL HotStarTaq DNA polymerase (Qiagen), 0.65 µL of 10× PCR buffer, and 0.35 µL of 25 mM MgCl₂ were mixed, and tertiary distilled water was added to a final volume of 5 µL. After reacting the mixture at 94°C for 15 minutes, the HotStarTaq polymerase was activated, followed by repeating the reaction of [94°C (20 sec)/56°C (30 sec)/72°C (60 sec)] 45 times and then reacting at 72°C for 3 minutes.

### B. Shrimp Alkaline Phosphatase (SAP) treatment

In order to deactivate remaining unused dNTP for each amplified product during multiplex PCR process, 0.2 µL of SAP buffer, 0.3 µL of SAP, and 1.5 µL of the tertiary distilled water were added to the amplified product of the multiplex PCR of reaction A., and reacted at 37°C and 85°C for 40 minutes and 10 minutes, respectively.

### C. Single Base Extension reaction

0.2 µL of 10× Plus iPLEX buffer, 0.1 µL of iPLEX termination mix, 1.2 µL of UEP mix (7 µM or 14 µM depending on the molecular weight), 0.02 µL of Sequenase, and 0.48 µL of tertiary distilled water were added to the product of reaction B above, and then reacted at 94°C for 30 seconds. The reaction of [94°C (5 sec)/ 52°C (5 sec)/ 80°C (5 sec)/ 52°C (5 sec)/ 80°C (5 sec)/ 52°C (5 sec)/ 80°C (5 sec)/ 52°C (5 sec)/ 80°C (5 sec)/ 52°C (5 sec)/ 80°C (5 sec)] was repeated 40 times, followed by incubating at 72°C for 3 minutes, thereby completing the reaction.

### (3) Genotyping Analysis

After aliquoting each of the above reactants to each spot of SpectroChip II (Sequenom), data was obtained using MALDI-ToF instrument (Sequenom) and was analyzed with Typer Analyzer program (Sequenom). The genotype of each SNP was identified by confirming changes in UEP molecular weights for each of 24 SNPs.

As a result, as shown in Fig. 3, in a case of using DNA isolated using a method of 100 µL of lysis buffer + PEG as a template, 150 genotypes out of a total of 216 genotypes were shown to have a clear peak pattern. Accordingly, it can be seen that the DNA extraction method using the bead method of the present invention enables high-quality DNA extraction for the most accurate SNP genotyping compared to other DNA extraction methods (QIAGEN, 100 µL of lysis buffer or 50 µL of lysis buffer).

### Example 7: Analysis of recovery ratio of the sample obtained by the genome DNA PREP method of Example 1

The recovery ratio of 100 ng of gDNA isolated from 5 different FFPE tissues using QIAamp DNA FFPE Tissue kit when re-extracted using QIAamp DNA FFPE Tissue kit, QIAquick PCR Purification kit, or the bead method (ASAN-Method) of the present invention was compared. Details of the experimental method are as follows:

### (1) QIAamp DNA FFPE Tissue kit

100 ng of gDNA purely isolated in 200 µL of an ATL solution was stirred, and was mixed well with 200 µL of each of an AL solution and 100% ethanol. The mixture was transferred to a QIAamp MinElute column placed in a 2 mL collection tube and was then centrifuged at 8,000 rpm for 1 minute.

The QIAamp MinElute column was transferred to a new 2 mL collection tube, followed by adding 500 µL of an AW1 solution and centrifuging at 8,000 rpm for 1 minute.

Then, the QIAamp MinElute column was transferred to a new 2 mL collection tube, followed by adding 500 µL of an AW2 solution and centrifuging at 8,000 rpm for 1 minute.

The QIAamp MinElute column was then transferred to a new 2 mL collection tube and was centrifuged at 14,000 rpm for 3 minutes.

After transferring the QIAamp MinElute column to a new 1.5 mL tube, and 30 µL of an ATE solution was aliquoted at the center of the column and was left to stand at room temperature for 1 minute. This was followed by centrifugation at 14,000 rpm for 1 minute.

The QIAamp MinElute column was removed, and DNA was obtained in a 1.5 ml tube. A concentration of the DNA was measured by PicoGreen. A final recovery ratio (the total amount obtained/100 ng) was calculated by multiplying the volume of the eluate by 30.

### (2) QIAquick PCR Purification kit

50 µL (5-fold greater volume) of a PB1 solution was added to 100 ng (10 µL) of the purely isolated DNA and was mixed well. Next, the mixture was transferred to a QIAamp MinElute column placed in a 2 mL collection tube and was then centrifuged at 8,000 rpm for 1 minute.

The QIAquick spin column was transferred to a new 2 mL collection tube, followed by adding 750 µL of a PE solution and centrifuging at 8,000 rpm for 1 minute.

Next, the QIAquick spin column was then transferred to a new 2 mL collection tube and was centrifuged at 14,000 rpm for 1 minute.

After transferring the QIAquick spin column was to a new 1.5 mL tube, 30 µL of an EB solution was aliquoted at the center of the column and was left to stand at room temperature for 1 minute, followed by centrifuging at 14,000 rpm for 1 minute.

The QIAquick spin column was removed, and DNA was obtained in a 1.5 mL tube. A concentration of the DNA was measured by PicoGreen. A final recovery ratio (the total amount obtained/100 ng) was calculated by multiplying the volume of the eluate by 30.

### (3) Bead method of the present invention

100 ng of the DNA purely isolated in 100 µL of the lysis buffer was mixed in a clean 1.5 mL tube, 100 µL of each of an AMPure XP solution and solution A (2.5 M NaCl, 20% PEG-8000) was added and mixed well.

The mixture was reacted at room temperature for 3 minutes and placed on the magnetic stand for 5 minutes. The remaining solution was discarded, with care being taken not to touch the beads at the bottom.

500 µL of 85% ethanol was added and then discarded after 30 seconds. This was repeated twice, and was left to stand at room temperature for 5 minutes to evaporate any remaining ethanol.

After transferring the mixture from the magnetic stand to a different location, 30 µL of an eluate was added and mixed well, and was then reacted at room temperature for 5 minutes.

The mixture was returned to the magnetic stand, and the beads were allowed to sink. While taking care not to touch the bead pellet, only the solution was transferred to a new 1.5 mL tube to obtain the extracted DNA. A concentration of the DNA was quantified with PicoGreen. A final recovery ratio (the total amount obtained/100 ng) was calculated by multiplying the volume of the eluate by 30.

As a result, when the bead method (ASAN-Method) was used, the highest recovery ratio was obtained as shown in Fig. 4. Two Qiagen products showed a difference, with the PCR purification kit showing a higher recovery ratio compared to the FFPE tissue kit. It is considered that the PCR purification kit is manufactured for the use of extracting DNA in a size of an amplified product, and thus enables one to more effectively obtain fragmented FFPE DNA. However, the bead method of the present invention shows a significantly higher recovery rate of FFPE DNA compared to the PCR purification kit, and is considered excellent in obtaining fragmented FFPE DNA.

### Example 8: Analysis of yield of the sample obtained by the genome DNA PREP method of Example 1

Yield of DNA extracted by the bead method developed in the present invention was confirmed using 1 to 5 identical FFPE blocks sliced in the same thickness of 6 µm(Fig. 5). As a result, it was confirmed that yield that can be obtained is about 5 µg based on PicoGreen quantitation under the condition of the present invention, in which 100 µL of lysis buffer, 100 µL of beads, and 100 µL of solution A were used.

### Example 9: Confirmation of reproducibility of the genome DNA PREP method of Example 1

Yield of the DNA and quality of the extracted DNA of each method was confirmed by a researcher from another institution by extracting DNA and calculating a quantitative value of ND and PG using the bead method of the present invention and the column method using MN kit.

DNA extracted by each method in electrophoresis was compared per reagent. Specifically, 2 µL of the final DNA extract was loaded on 1.5% agarose gel, and electrophoresis was performed at 100 V for 25 minutes. An electrophoresis image was analyzed using GelDoc imaging system (Biorad). To determine a size of the DNA, a 100 bp DNA ladder was also used in electrophoresis.

As a result, it was confirmed that the DNA extracted by the bead method showed a much higher intensity band that that extracted by the MN kit (Fig. 6).

Additionally, yield of the DNA extracted using the beads was two- to three-fold higher (Fig. 7; left and center). In particular, in comparison with the yields of DNA extracted by the bead method and that extracted by the MN kit, the difference in PicoGreen quantitative values was larger than that of NanoDrop quantitative values, and this suggests that the DNA extracted using the beads included dsDNA, which is an intact form, in a larger amount.

Further, through the analysis of an ND/PG ratio, which enables an examination of DNA quality, it was confirmed the DNA extracted by the bead method showed a relatively low ND/PG ratio compared to that extracted by the MN kit (Fig. 7; right).

In conclusion, the method of the present invention for isolating a nucleic acid from an FFPE tissue using magnetic beads enables one to obtain high-quality DNA from not only a FFPE tissue which has recently been manufactured but also an FFPE tissue aged at least 10 years in high yield, compared to other DNA extraction methods.

The described embodiments are to be considered in all respects only as illustrative and not restrictive. The scope of the present invention is therefore indicated by the appended claims rather than by the foregoing description.

## Claims

1. A method for isolating a nucleic acid from a formalin-fixed, paraffin-embedded (FFPE) tissue, comprising:
(a) lysing FFPE tissue fragments in 90 µl or 200 µl lysis buffer comprising a chelating agent, an ionic surfactant, and Tris-CI, and 10 µl proteinase K, to prepare a separated sample;
(b) adding, to the separated sample, 100 µl solution which comprises 2.5 M of sodium chloride, and 20% (w/v) polyethylene glycol (PEG-8000), and 100 µl magnetic bead solution; and
(c) isolating the nucleic acid from the magnetic beads after obtaining the magnetic beads from the sample comprising the same.

2. The method of claim 1, wherein the lysis buffer comprises the chelating agent in a concentration of 10 mM to 100 mM; the ionic surfactant in a concentration of 0.1% (w/v) to 5% (w/v); and the Tris-CI in a concentration of 10 mM to 100 mM.

3. The method of claim 1, wherein the chelating agent is selected from the group consisting of diethylenetriaminepentaacetic acid (DTPA), ethylenediaminetetraacetic acid (EDTA), ethylene glycol tetraacetic acid (EGTA), and *N*,*N*-bis(carboxymethyl)-glycine (NTA).

4. The method of claim 1, wherein the ionic surfactant is selected from the group consisting of sodium dodecyl sulfate (SDS), ammonium lauryl sulfate, sodium lauryl ether sulfate (SLES), sodium myreth sulfate, dioctyl sodium sulfosuccinate, perfluorooctanesulfonate (PFOS), perfluorobutanesulfonate, linear alkylbenzene sulfonates (LABs), sodium lauroyl sarcosinate, perfluorononanoate (PFOA), and perfluorooctanoate (PFO).

5. The method of claim 1, wherein the lysis buffer comprises EDTA, SDS, and Tris-Cl.

## Patentansprüche

1. Verfahren zum Isolieren einer Nukleinsäure aus Formalinfixiertem Paraffin-eingebettetem Gewebe (FFPE), umfassend:
(a) Lysieren von FFPE-Gewebefragmenten in 90 µl oder 200 µl Lyse-Puffer, umfassend einen Chelatbildner, ein ionisches Tensid und Tris-Cl, sowie 10 µl Proteinase K, um eine separierte Probe herzustellen;
(b) Zugeben zu der separierten Probe von 100 µl Lösung, die 2,5 M Natriumchlorid und 20 % (w/v) Polyethylenglykol (PEG-8000) umfasst, sowie 100 µl Magnetperlen-Lösung; und
(c) Isolieren der Nukleinsäure von den Magnetperlen nach dem Gewinnen der Magnetperlen aus der Probe, welche dieselben umfasst.

2. Verfahren nach Anspruch 1, wobei der Lyse-Puffer den Chelatbildner in einer Konzentration von 10 mM bis 100 mM; das ionische Tensid in einer Konzentration von 0,1 % (w/v) bis 5 % (w/v); und das Tris-Cl in einer Konzentration von 10 mM bis 100 mM umfasst.

3. Verfahren nach Anspruch 1, wobei der Chelatbildner ausgewählt ist aus der Gruppe bestehend aus Diethylentriaminpenta-Essigsäure (DTPA), Ethylendiamintetra-Essigsäure (EDTA), Ethylenglykoltetra-Essigsäure (EGTA), und N,N-Bis(carboxymethyl)-glycin (NTA).

4. Verfahren nach Anspruch 1, wobei das ionische Tensid ausgewählt ist aus der Gruppe bestehend aus Natriumdodecylsulfat (SDS), Ammoniumlaurylsulfat, Natriumlauryl-Ethersulfat (SLES), Natriummyrethsulfat, Dioctyl-Natriumsulfosuccinat, Perfluoroctansulfonat (PFOS), Perfluorbutansulfonat, linearen Alkylbenzolsulfonaten (LABs), Natriumlauroylsarcosinat, Perfluornonanoat (PFOA) und Perfluoroctanoat (PFO).

5. Verfahren nach Anspruch 1, wobei der Lyse-Puffer EDTA, SDS und Tris-Cl umfasst.

## Revendications

1. Méthode permettant d'isoler un acide nucléique provenant d'un tissu incorporé dans de la paraffine et fixé au formol (FFPE), comprenant :
(a) la lyse de fragments de tissu FFPE dans 90 µl ou 200 µl de tampon de lyse comprenant un agent chélateur, un tensioactif ionique, et du Tri-Cl, et 10 µl de protéinase K, pour préparer un échantillon distinct ;
(b) l'ajout, à l'échantillon distinct, de 100 µl d'une solution qui comprend 2,5 M de chlorure de sodium, et 20 % (p/v) de polyéthylène glycol (PEG-8000) et 100 µl d'une solution de billes magnétiques ; et
(c) l'isolement de l'acide nucléique des billes magnétiques après obtention des billes magnétiques à partir de l'échantillon comprenant ces billes.

2. Méthode selon la revendication 1, dans laquelle le tampon de lyse comprend l'agent chélateur en une concentration de 10 mM à 100 mM ; le tensioactif ionique en une concentration de 0,1 % (p/v) à 5 % (p/v) ; et le Tri-Cl en une concentration de 10 mM à 100 mM.

3. Méthode selon la revendication 1, dans laquelle l'agent chélateur est choisi dans le groupe constitué par l'acide diéthylènetriaminepentaacétique (DTPA), l'acide éthylènediaminetétraacétique (EDTA), l'acide éthylène glycol tétraacétique (EGTA) et le N,N-bis(carboxyméthyl)-glycine (NTA).

4. Méthode selon la revendication 1, dans laquelle le tensioactif ionique est choisi dans le groupe constitué par le dodécylsulfate de sodium (SDS), le laurylsulfate d'ammonium, le lauryléthersulfate de sodium (SLES), le myrethsulfate de sodium, le sulfosuccinate de sodium dioctylique, le perfluorooctanesulfonate (PFOS), le perfluorobutanesulfonate, les sulfonates d'alkylbenzène linéaires (LAB), le lauroylsarcosinate de sodium, le perfluorononanoate (PFOA) et le perfluorooctanoate (PFO).

5. Méthode selon la revendication 1, dans laquelle le tampon de lyse comprend de l'EDTA, du SDS et du Tris-Cl.
